# FASCICULE DE BREVET EUROPEEN

(11) **EP 4 358 827 B1**
(45) Date de publication et mention de la délivrance du brevet: **06.08.2025**
(21) Numéro de dépôt: 22744498.1
(22) Date de dépôt: 22.06.2022
(51) Int. Cl.: A61B 5/00, A61B 5/0205, A61B 5/021, A61B 5/085, A61B 5/087, A61B 5/12, A61B 5/117, A61B 5/1455, A61B 5/0537

(54) **DISPOSITIF DE MESURES PHYSIOLOGIQUES ET PROCEDE D'INSTALLATION ASSOCIE**
VORRICHTUNG FÜR PHYSIOLOGISCHE MESSUNGEN UND ENTSPRECHENDES EINSTELLVERFAHREN
DEVICE FOR PHYSIOLOGICAL MEASUREMENTS, AND ASSOCIATED SET-UP METHOD

(30) Priorité: 23.06.2021 FR 2106678
(43) Date de publication de la demande: 01.05.2024
(73) Titulaire: Matrix, 06200 Nice (FR)
(72) Inventeur: COUTARD, Patrice, 69660 COLLONGES AU MONT D'OR (FR)
(74) Mandataire: A.P.I. Conseil
(86) Numéro de dépôt international: PCT/FR2022/051215
(87) Numéro de publication internationale: WO 2022/269192

(56) Documents cités:
- EP-A1- 3 153 145
- CN-B- 104 771 154
- CN-U- 205 054 771
- FR-A1- 2 898 483
- US-A1- 2020 368 090
- US-A1- 2021 153 753
- US-B2- 10 445 463

## Description

### Domaine de l'invention

La présente invention concerne le domaine technique de la prise de mesures physiologiques de manière autonome ou semi-autonome, c'est-à-dire avec ou sans l'assistance d'une personne physique, présente ou distante. L'invention permet la réalisation d'un autodiagnostic médicale et/ou la mise en œuvre d'une téléconsultation médicale en interagissant avec un praticien.

Plus particulièrement, l'invention vise un dispositif de mesures physiologiques permettant l'intégration d'un grand nombre de capteurs dans un environnement réduit. L'invention vise également le procédé d'installation d'un tel dispositif.

Les faibles dimensions du dispositif de mesures physiologiques de l'invention permettent d'envisager des multiples applications, par exemple pour former une salle de téléconsultation dans un lieu accueillant du public, tel qu'une mairie, ou un espace privé, tel qu'une maison de retraite, et faciliter l'accessibilité à une téléconsultation. En outre, les faibles dimensions du dispositif de mesures physiologiques peuvent permettre son intégration dans un drone, un véhicule terrestre, un navire ou tout autre véhicule.

### Etat antérieur de la technique

Le développement croissant des systèmes de téléconsultation permet de faciliter l'accès à la médecine. Par exemple, les systèmes de téléconsultation permettent de lutter contre les déserts médicaux et permettent un accès facilité à la médecine, notamment la médecine d'urgence.

Pour réaliser des téléconsultations médicales, il existe un grand nombre d'applications mobiles permettant à un praticien de dialoguer avec un patient. Ces applications sont limitées car elles ne permettent pas d'obtenir des mesures physiologiques contrôlées pour permettre au praticien d'établir un diagnostic fiable.

Il existe également des bornes de téléconsultation ou d'autodiagnostic. Ces bornes intègrent classiquement un écran et une caméra permettant d'interagir avec un praticien, ou un logiciel permettant de guider un patient pour lui permettre d'effectuer un autodiagnostic. De part et d'autre de cet écran, le patient peut interagir avec un ensemble de capteurs, tels qu'un dermatoscope, un otoscope, un oxymètre, un stéthoscope, un tensiomètre ou un thermomètre.

Lors d'une téléconsultation ou d'un autodiagnostic, le praticien ou le logiciel peuvent demander au patient d'utiliser plusieurs capteurs successivement pour établir un diagnostic. Cette phase de diagnostic peut durer plusieurs longues minutes au cours de laquelle le patient est souvent assis sur une chaise devant la borne et il doit se déplacer périodiquement pour atteindre l'un ou l'autre des capteurs.

En variante, pour faciliter l'accessibilité aux capteurs et améliorer la confidentialité, il existe également des cabines de téléconsultation, tel que le produit de « AI POD » du Demandeur ou tel que décrit dans le document EP 3 420 892. Ces cabines intègrent classiquement un siège sur lequel le patient est destiné à s'assoir et un écran placé en face du siège.

Plus particulièrement, ces cabines de téléconsultation visent à maximiser le nombre de mesures pouvant être effectuées en un minimum de temps, facteur de la qualité de la téléconsultation ou de l'autodiagnostic. Par exemple, le produit « AI POD » précité permet actuellement l'acquisition de 26 mesures physiologiques en 6 minutes.

Pour obtenir ce type de vitesse de prise de mesures, les cabines proposent classiquement de disposer les capteurs physiologiques autour du siège sur lequel le patient est destiné à prendre place, de sorte à faciliter l'accès à chaque capteur. Pour ce faire, les cabines de téléconsultation utilisent généralement une coque supportant le siège et l'écran placé en face du siège. Cette coque est formée par une double paroi intégrant une unité centrale sur laquelle l'ensemble des capteurs physiologiques sont connectés. Ainsi, les capteurs physiologiques sont placés autour du siège de la cabine et les connecteurs de ces capteurs pénètrent à travers des ouvertures de la coque avant d'être connectés sur l'unité centrale.

Le document US 2021/153753 A1 décrit une autre cabine interactive de téléconsultation médicale, comprenant une pluralité de capteurs.

Cependant, les solutions existantes ne permettent pas d'obtenir une cabine de faible encombrement puisque la coque est nécessairement assez volumineuse pour supporter le siège, l'écran et l'ensemble des capteurs, et intégrer l'unité centrale et les différents connecteurs des capteurs.

Il s'ensuit que ces cabines présentent classiquement des dimensions minimum d'environ 3 mètres de longueur sur 1.5 mètre de largeur. Ces dimensions imposantes ne permettent pas d'intégrer ces cabines dans tous les espaces. Par exemple, ces cabines ne peuvent pas passer à travers une porte standard, de largeur inférieure à 1 mètre.

Il en résulte que les solutions existantes présentent soit une qualité de prise de mesure et un niveau de confidentialité limité, cas des bornes, soit un encombrement important et des difficultés d'installation corrélatives, cas des cabines.

Le problème technique de l'invention est d'obtenir un dispositif de mesures physiologiques permettant un accès rapide à un grand nombre de capteurs avec une capacité d'installation améliorée.

### Exposé de l'invention

Pour répondre à ce problème technique, l'invention propose un dispositif de mesures physiologiques avec une coque comportant au moins deux parties amovibles : une première partie supportant le siège et un grand nombre de capteurs et intégrant l'unité centrale ; et une seconde partie supportant principalement un capteur de pression artérielle.

L'écran peut être intégré dans une troisième partie de la coque de sorte que l'assemblage des trois parties de la coque forme une cabine avec l'écran placé en face du siège. En variante, l'écran peut être fixé en face du siège sans être lié mécaniquement au siège, typiquement pour aménager un espace de téléconsultation ou d'autodiagnostic.

Par exemple, pour aménager une salle de téléconsultation dans un espace public ou privé existant, l'écran peut être fixé sur un mur. La coque est ensuite transportée et installée devant l'écran en déplaçant les deux parties de la coque successivement et en passant au travers de portes standards.

La découverte de la possibilité de dissocier les éléments de la coque pour obtenir une coque amovible en au moins deux parties est particulièrement surprenante compte tenu de l'encombrement des capteurs et des connecteurs des cabines existantes. Pour garantir à la fois la facilité d'installation et l'accessibilité aux différents capteurs, il a été identifié que la coque devait être scindée en deux parties bien différentes, tant par leurs formes que par le nombre et le type de capteurs qu'elles supportent.

A cet effet, selon un premier aspect, l'invention concerne un dispositif de téléconsultation comportant : une coque supportant un siège et formant des accoudoirs gauche et droit ; une unité centrale intégrée à l'intérieur de la coque ; et un ensemble de capteurs disposés au niveau des accoudoirs gauche et droit, les capteurs étant connectés sur l'unité centrale à travers des ouvertures de la coque.

L'invention se caractérise en ce que la coque est réalisée en deux parties amovibles :
- une première partie formant l'accoudoir droit, supportant le siège et au moins quatre autre capteurs et intégrant l'unité centrale ; et
- une seconde partie formant l'accoudoir gauche et supportant au moins un capteur de pression artérielle de sorte que l'installation du dispositif de téléconsultation médicale puisse être réalisée en solidarisant les deux parties de la coque et en reliant l'au moins un capteur de pression artérielle sur l'unité centrale.

Au sens de l'invention, l'accoudoir gauche correspond à l'accoudoir présent à gauche du siège et permettant au patient assis sur le siège de reposer son bras gauche lorsque les deux parties de la coque sont assemblées. De même, l'accoudoir droit correspond à l'accoudoir présent à droite du siège et permettant au patient assis sur le siège de reposer son bras droit lorsque les deux parties de la coque sont assemblées.

La solution de l'invention consistant à positionner le capteur de pression artérielle au niveau de l'accoudoir de gauche n'est pas anodine puisque les mesures de pression artérielle sont plus précises lorsqu'elles sont réalisées sur le bras gauche, c'est-à-dire sur le bras le plus proche du cœur. En outre, les capteurs de pression artérielle permettant d'obtenir des mesures précises sont souvent plus volumineux que les autres capteurs pouvant être disposés au niveau des accoudoirs, tels que les dermatoscopes, les otoscopes, les oxymètres, les stéthoscopes, les tensiomètres ou les thermomètres.

Ainsi, en supportant principalement le capteur de pression artérielle sur la seconde partie amovible formant l'accoudoir gauche, il est possible de désolidariser des éléments volumineux, tels que l'accoudoir gauche et le capteur de pression artérielle, tout en limitant la complexité d'assemblage des deux parties de la coque. En effet, la désolidarisation de la coque en plusieurs parties induit une complexité lors de l'assemblage de la coque, notamment pour connecter les capteurs présents dans la partie qui n'intègre pas l'unité centrale car ces capteurs ne peuvent pas être connectés sur l'unité central avant le transport.

Le positionnement du capteur le plus volumineux pouvant être disposé au niveau des accoudoirs dans la partie n'intégrant pas l'unité centrale a permis de faciliter le transport et l'installation tout en limitant la complexité lors de l'assemblage de la coque. En effet, il est désormais possible d'obtenir deux parties de la coque dont les dimensions sont inférieures à 1m, permettant ainsi le passage des différentes parties du dispositif de téléconsultation médicale à travers des portes standards.

Par ailleurs, le capteur de pression artérielle n'est pas le capteur le plus volumineux pouvant être intégré dans le dispositif de téléconsultation médicale car il peut être recherché d'intégrer une balance ou encore un capteur de hauteur du patient. De préférence, ces capteurs sont également supportés par la première partie formant l'accoudoir droit de sorte qu'ils puissent être connectés à l'unité centrale avant le transport et l'installation de la première partie.

Ainsi, l'invention ne propose pas d'intégrer simplement le capteur le plus volumineux dans une partie amovible mais plus spécifiquement d'intégrer le capteur le plus volumineux pouvant être placé au niveau d'un accoudoir de sorte à désolidariser à la fois la structure formant l'accoudoir et ce capteur. Bien entendu, d'autres capteurs peuvent également être placés sur la seconde partie formant l'accoudoir gauche en plus du capteur de pression artérielle. Pour faciliter la connexion de plusieurs capteurs placés sur la partie amovible de celle intégrant l'unité centrale, il peut être formé un faisceau de connexion pour l'ensemble de ces capteurs afin de faciliter leurs connexions sur l'unité centrale.

Pour obtenir un grand nombre de mesures et faciliter l'installation, la première partie formant l'accoudoir droit supporte un capteur de composition corporelle, un oxymètre, un audiomètre, un électrocardiogramme, un échographe, un otoscope, un stéthoscope, un inspiromètre, un dermatoscope, un échographe et un scanner de vision.

L'oxymètre permet classiquement d'obtenir le taux de saturation d'oxygène dans le sang. En outre, l'unité centrale peut intégrer les traitements numériques, par exemple permettant d'extraire le rythme cardiaque ou encore la rigidité artérielle à partir de la mesure de l'oxymètre.

De préférence, le capteur de composition corporelle correspond un capteur pied/main permettant d'obtenir des informations particulièrement fiables sur l'ensemble du corps, non seulement au niveau du taux de masse graisseuse du patient mais aussi de sa masse musculaire, de son hydratation intracellulaire extracellulaire ...

Outre les capteurs intégrés dans la coque, des capteurs peuvent être intégrés au niveau de l'écran ou dans une borne d'accès à un espace intégrant le siège et l'écran.

Dans ce dernier exemple, le dispositif de téléconsultation comporte également une borne d'accès, destinée à être fixée à l'entrée d'un espace dans lequel l'écran et le siège sont installés, la borne comportant des moyens d'identification et un capteur de température.

Ce mode de réalisation permet de prendre la température du patient, par exemple au moyen d'un capteur infrarouge, lors de l'accès du patient à l'espace de téléconsultation. Par exemple, l'accès à l'espace de téléconsultation peut être contrôlé par une carte d'accès, des moyens de reconnaissance biométriques ou tout autre moyen d'identification.

L'ensemble des capteurs intégrés dans la coque, la borne d'accès et éventuellement l'écran permet de former une salle de téléconsultation à partir d'une salle existante.

Pour ce faire, selon un second aspect, le procédé d'installation du dispositif de téléconsultation médicale selon le premier aspect de l'invention comporte les étapes suivantes :
- déplacement de la première partie de la coque dans une salle ;
- déplacement de la seconde partie de la coque dans la salle ;
- fixation de la seconde partie sur la première partie de la coque ;
- connexion de l'au moins un capteur de pression artérielle sur l'unité centrale ;
- intégration d'un écran dans la salle ; et
- connexion de l'écran avec l'unité centrale.

De préférence, pour fixer la seconde partie de la coque sur la première partie de celle-ci, la coque comporte des détrompeurs mécaniques coopérant entre les deux parties de la coque.

En outre, pour assurer une fixation des deux parties de la coque, une trappe d'accès peut être ménagée derrière le siège de la première partie de la coque. Cette trappe d'accès permet d'accéder à l'unité centrale pour connecter l'au moins un capteur de pression artérielle, et elle permet également de boulonner les deux parties de la coque pour assurer une fixation pérenne.

### Brève description des figures

L'invention sera bien comprise à la lecture de la description qui suit, dont les détails sont donnés uniquement à titre d'exemple, et développée en relation avec les figures annexées, dans lesquelles des références identiques se rapportent à des éléments identiques :
La figure illustre une vue en perspective éclatée d'un dispositif de téléconsultation médicale selon un premier mode de réalisation de l'invention ;
La figure 2 illustre une vue en coupe de côté du dispositif de téléconsultation médicale de la figure 1 ;
La figure 3 illustre une vue en perspective d'un accès de maintenance ménagé derrière un siège du dispositif de téléconsultation médicale de la figure 1 ;
La figure 4 illustre une vue de face d'un dispositif de téléconsultation médicale selon un second mode de réalisation de l'invention ;
La figure 5 illustre une vue de côté du dispositif de téléconsultation médicale de la figure 4 ; et
La figure 6 illustre une vue en perspective de dessus du dispositif de téléconsultation médicale de la figure 4.

### Description détaillée de l'invention

La description qui suit illustre deux modes de réalisation distincts : un premier mode de réalisation d'un dispositif de téléconsultation **10a** utilisé pour aménager un espace clos, tel qu'une salle dédiée à la téléconsultation dans une mairie ; et un second mode de réalisation d'un dispositif de téléconsultation **10b** prenant la forme d'une cabine. Dans ces deux modes de réalisation, une coque **11a-11b** est formée autour d'un siège **12** et présente une double cloison de sorte à supporter et à intégrer les éléments électriques et électroniques d'alimentation et de commande d'un ensemble de capteurs **16a-16h.**

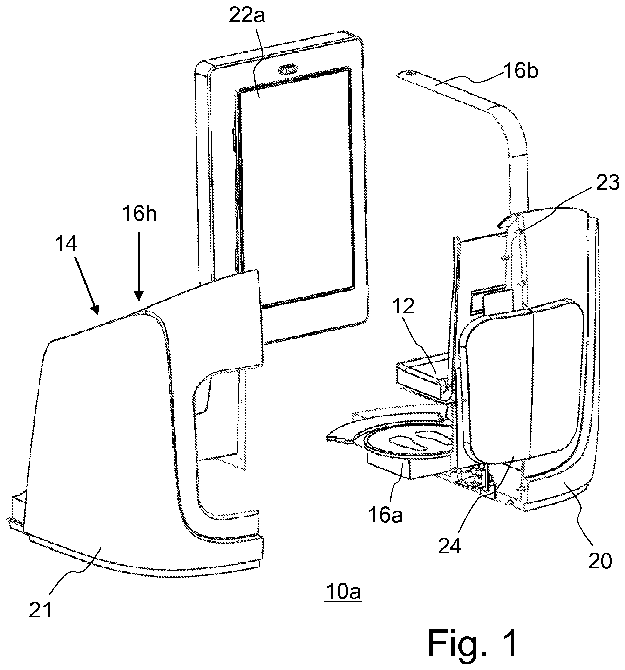

Dans le premier mode de réalisation, illustré sur les figures 1 à 3, la coque **11a** est réalisée en deux parties **20-21** amovibles alors que l'écran **22a** est positionné en face d'un siège **12** intégré par la première partie **20.** Selon l'invention, la première partie **20** forme l'accoudoir droit **13** et supporte le siège **12** alors que le seconde partie **21** forme l'accoudoir gauche **14.**

En outre, un capteur de pression artérielle **16h** est disposé sur l'accoudoir gauche **14** de la seconde partie **21** afin de mesurer la pression artérielle du patient lorsque celui-ci est assis sur le siège **12.**

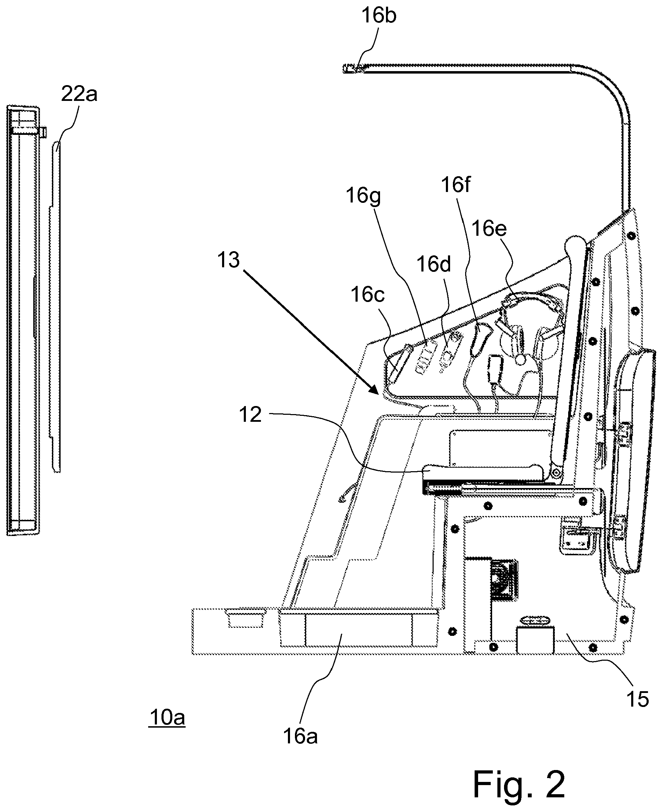

D'autres capteurs peuvent également être disposés au niveau de l'accoudoir gauche **14** afin que le patient puisse facilement les atteindre. De préférence, la plus grande partie des capteurs **16a-16g** sont disposés au niveau de l'accoudoir droit **13,** tel qu'illustré sur la figure 2. Par exemple, plusieurs capteurs peuvent être accessibles au-dessus de l'accoudoir droit **13,** tels qu'un oxymètre **16d,** un audiomètre **16e,** un électrocardiogramme **16f,** un échographe **16g,** un otoscope, un stéthoscope, un inspiromètre, un dermatoscope, un échographe et un scanner de vision. Bien entendu, d'autres types de capteurs peuvent être utilisés sans changer l'invention.

Tel qu'illustré sur la figure 2, le sol du dispositif de téléconsultation **10a** peut être formé par une balance **16a** fixée sur la première partie **20** alors que la partie supérieure du dispositif de téléconsultation **10a** peut être instrumentée par un capteur de hauteur **16b** du patient, également fixé sur la première partie **20.** Un capteur de composition corporelle **16c** est préférentiellement intégré dans la première partie **20,** par exemple un capteur pied/main.

Selon l'invention au moins quatre capteurs **16a-16g** sont supportés par la première partie **20.** En effet, il est préférable d'intégrer les capteurs dans la première partie **20** car cette partie **20** intègre également l'unité centrale **15** sur laquelle les capteurs **16a-16h** sont connectés. Plus précisément, l'unité centrale **15** peut être placée sous le siège **12** derrière une trappe d'accès **24** ménagée derrière le siège **12** dans la première partie **20** de la coque.

Par exemple, cette trappe d'accès **24** peut être montée sur des charnières fixées sur la première partie **20** et intégrer un mécanisme de verrouillage.

L'unité centrale **15** peut correspondre à un ordinateur associé à une carte de connexion des différents capteurs **16a-16h.** Par exemple, un routeur de type USB, pour bus série universelle selon la littérature anglo-saxonne « *Universal Serial Bus* », peut être connecté sur l'ordinateur pour alimenter et réceptionner les données des différents capteurs **16a-16h.** Pour ce faire, les capteurs **16a-16h** sont connectés sur l'unité centrale au moyen de câbles USB pénétrant dans la double cloison par des ouvertures de la coque **11a.**

En variante, d'autres types de connexion peuvent être utilisées en fonction des capteurs **16a-16h** utilisés.

Outre ces connecteurs filaires avec les capteurs **16a-16h,** l'unité centrale **15** est également connectée avec un écran **22a,** avec ou sans fil. Dans le premier mode de réalisation, l'écran **22a** est fixé en face du siège **12** sans être lié mécaniquement au siège **12.** L'unité centrale **15** peut être connectée avec une borne d'accès fixée à l'entrée de l'espace dans lequel l'écran **22a-22b** et le siège **12** sont installés.

Dans le premier mode de réalisation, la borne d'accès peut être disposée à l'entrée d'une salle dans laquelle le siège **12** et l'écran **22a** sont installés et intégrer des moyens d'identification et un capteur de température.

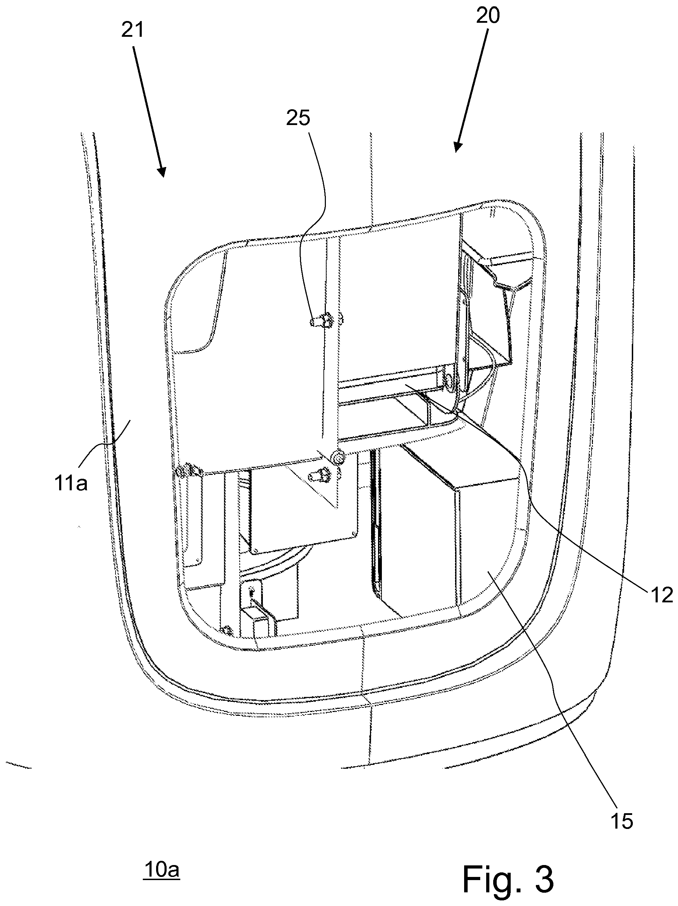

Pour obtenir la formation de la coque, la seconde partie **21** est fixée sur la première partie **20.** Pour ce faire, chaque paroi de la coque **11a** est pourvue de détrompeurs mécaniques **23** au niveau de la zone de liaison prédéterminée de la coque **11a.** La fixation des deux parties **20-21** de la coque **11a** peut également être assurée par des moyens de solidarisation de type vis/écrou **25.** De préférence, des plaques fixées sur chaque partie **20-21** sont disposées en regard l'une de l'autre avec des alésages coaxiaux de sorte à insérer les moyens de solidarisation de type vis/écrou **25** dans ces alésages. Tel qu'illustré sur la figure 3, ces alésages peuvent être accessibles dans la trappe d'accès **24.**

Outre la fixation des moyens de solidarisation de type vis/écrou **25,** la trappe d'accès **24** permet également de connecter le capteur de pression artérielle **16h** sur l'unité centrale **15** après fixation des deux parties **20-21** de la coque **11a.** Ainsi, l'unité centrale **15** est accessible depuis la trappe d'accès **24,** permettant ainsi d'effectuer des opérations de maintenance sur l'unité centrale **15.**

Outre l'unité centrale **15,** la double paroi formée par la coque **11a** permet également d'intégrer les composants électriques permettant l'alimentation du dispositif de téléconsultation **10a** et les connecteurs réseaux.

En effet, l'unité centrale **15** est préférentiellement également connectée au réseau Internet pour interagir avec un serveur informatique permettant d'assurer une communication sécurisée entre le dispositif de téléconsultation **10a** et un praticien. Ce serveur informatique peut également transmettre les informations d'authentification du patient pour autoriser son accès au dispositif de téléconsultation **10a** et déployer les mises à jour éventuelles du dispositif de téléconsultation **10a.**

Lorsque le patient est autorisé à utiliser le dispositif de téléconsultation **10a,** l'unité centrale **15** communique avec le serveur et guide le patient pour la prise de mesures physiologiques de manière autonome ou semi-autonome, c'est-à-dire avec ou sans l'assistance d'une personne physique, présente ou distante.

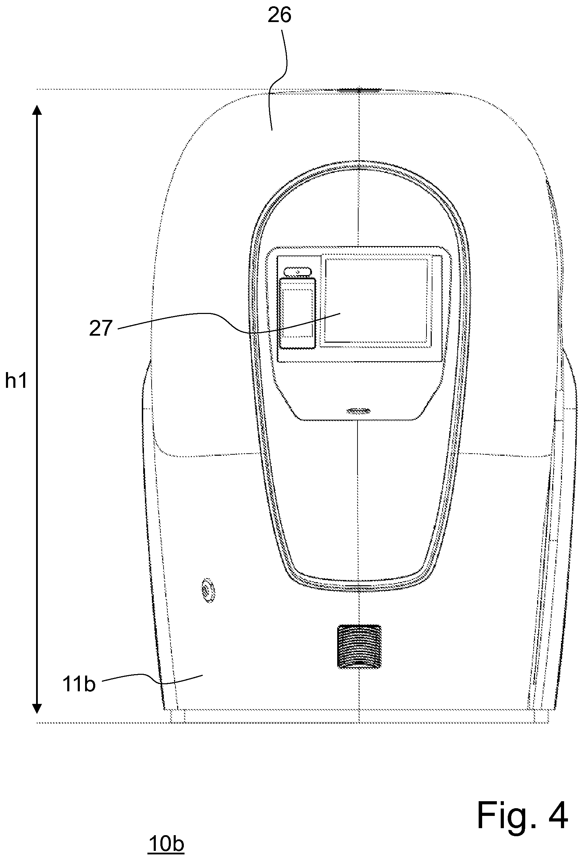

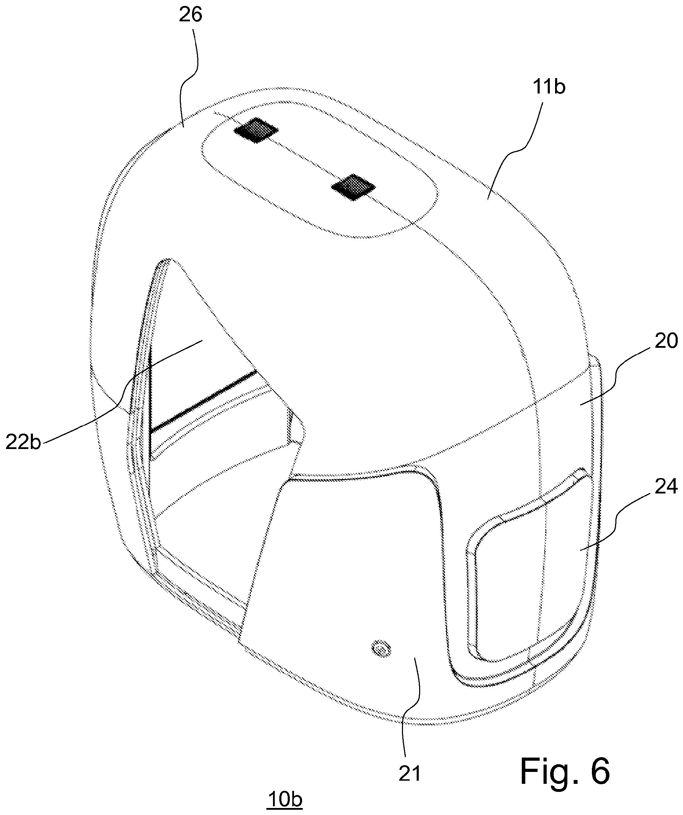

Dans le second mode de réalisation, illustré sur les figures 4 à 6, la coque **11b** reprend les deux parties **20-21** du premier mode de réalisation sur lesquelles sont rapportées une troisième partie **26** intégrant un écran **22b** de sorte que l'assemblage des trois parties **20, 21, 26** de la coque **11b** forme une cabine avec l'écran placé en face du siège **12.**

Cette troisième partie **26** peut être rapportée et fixée avec tous les moyens mécaniques connus. En outre, l'écran **22b** peut être connecté en filaire sur l'unité centrale **15.**

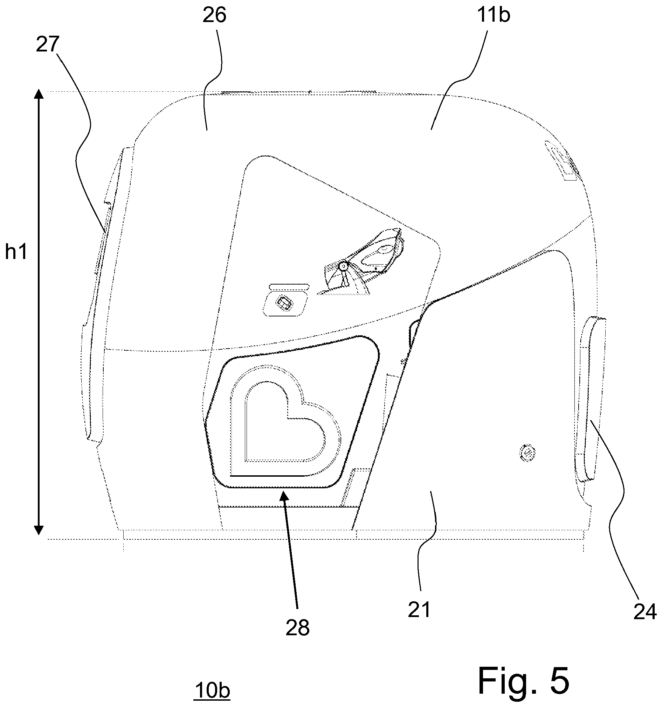

Dans le second mode de réalisation, tel qu'illustré sur les figures 4 et 5, la structure de la cabine garantie la confidentialité des échanges au moyen d'une porte **28** amovible préférentiellement fermé lorsque le patient est installé sur le siège **12.** Ainsi, la borne d'accès **27** peut être disposée directement sur la troisième partie **26** de la coque **11b.**

L'invention permet d'obtenir un dispositif de téléconsultation médicale **10a-10b** avec un siège **12** autour duquel un grand nombre de capteurs **16a-16h** peuvent être disposés et facilement accessibles. Le siège **12** et sa coque **11a** peut présenter des dimensions de l'ordre de 1500 à 2000 mm de longueur sur 1300 à 1800 mm de largeur dans le cas du premier mode de réalisation. Dans le second mode de réalisation, la cabine peut présenter des dimensions de l'ordre de 2300 à 3000 mm de longueur sur 1300 à 1800 mm de largeur et 2000 à 2500 mm de hauteur **h1.**

Malgré ces dimensions importantes, le montage de la coque **11a-11b** en plusieurs parties amovibles **20, 21, 26** permet de faciliter l'installation du dispositif de téléconsultation médicale **10a-10b.** En outre, la proximité des différents capteurs **16a-16h** permet l'acquisition de 26 mesures physiologiques en 6 minutes.

## Revendications

1. Dispositif de téléconsultation médicale (10a-10b) comportant :
- une coque (11a-11b) supportant un siège (12) et formant des accoudoirs gauche (14) et droit (13) ;
- une unité centrale (15) intégrée à l'intérieur de la coque (11a-11b) ; et
- un ensemble de capteurs (16a-16h) disposés au niveau des accoudoirs gauche (14) et droit (13), les capteurs (16a-16h) étant connectés sur l'unité centrale (15) à travers des ouvertures de la coque (11a-11b) ;
***caractérisé* en ce que** la coque (11a-11b) est réalisée en deux parties amovibles (20-21) :
- une première partie (20) formant l'accoudoir droit (13), supportant le siège (12) et au moins quatre autre capteurs (16a-16g) et intégrant l'unité centrale (15) ; et
- une seconde partie (21) formant l'accoudoir gauche (14) et supportant au moins un capteur de pression artérielle (16h) de sorte que l'installation du dispositif de téléconsultation médicale puisse être réalisée en solidarisant les deux parties (20-21) de la coque (11a-11b) et en reliant l'au moins un capteur de pression artérielle (16h) sur l'unité centrale (15).

2. Dispositif de téléconsultation médicale selon la revendication 1, ***dans lequel*** le dispositif comporte également un écran (22a) destiné à être fixé en face du siège (12) sans être lié mécaniquement au siège (12).

3. Dispositif de téléconsultation médicale selon la revendication 1, ***dans lequel*** la coque (11a-11b) comporte également une troisième partie (26) intégrant un écran (22b) de sorte que l'assemblage des trois parties (20, 21, 26) de la coque (11a-11b) forme une cabine avec l'écran placé en face du siège (12).

4. Dispositif de téléconsultation médicale selon l'une des revendications 1 à 3, ***dans lequel*** la première partie (20) formant l'accoudoir droit (13) supporte une balance (16a) et/ou un capteur de hauteur (16b).

5. Dispositif de téléconsultation médicale selon l'une des revendications 1 à 4, ***dans lequel*** la première partie (20) formant l'accoudoir droit (13) supporte un capteur de composition corporelle (16c), un oxymètre (16d), un audiomètre (16e), un électrocardiogramme (16f), un échographe (16g), un otoscope, un stéthoscope, un inspiromètre, un dermatoscope, un échographe et un scanner de vision.

6. Dispositif de téléconsultation médicale selon la revendication 5, ***dans lequel*** le capteur de composition corporelle (16c) correspond un capteur pied/main

7. Dispositif de téléconsultation médicale selon la revendication 2, ***dans lequel*** le dispositif de téléconsultation (10a-10b) comporte également une borne d'accès (27), destinée à être fixée à l'entrée d'un espace dans lequel l'écran (22a-22b) et le siège (12) sont installés, la borne d'accès (27) comportant des moyens d'identification et un capteur de température.

8. Dispositif de téléconsultation médicale selon l'une des revendications 1 à 7, ***dans lequel*** la coque comporte des détrompeurs mécaniques (23) coopérant entre les deux parties (20-21) de la coque (11a-11b).

9. Dispositif de téléconsultation médicale selon l'une des revendications 1 à 8, ***dans lequel*** la coque (11a-11b) comporte une trappe d'accès (24) ménagée derrière le siège (12) de la première partie (20) de la coque (11a-11b).

10. Procédé d'installation du dispositif de téléconsultation médicale selon l'une des revendications 1 à 9, le procédé comportant les étapes suivantes :
- déplacement de la première partie (20) de la coque (11a-11b) dans une salle ;
- déplacement de la seconde partie (21) de la coque (11a-11b) dans la salle ;
- fixation de la seconde partie (21) sur la première partie (20) de la coque (11a-11b) ;
- connexion de l'au moins un capteur de pression artérielle (16h) sur l'unité centrale (15) ;
- intégration d'un écran (22a-22b) dans la salle ; et
- connexion de l'écran (22a-22b) avec l'unité centrale (15).

## Patentansprüche

1. Vorrichtung für medizinische Fernkonsultation (10a-10b), aufweisend:
- eine Schale (11a-11b), die einen Sitz (12) trägt und die linke (14) und rechte (13) Armlehne bildet;
- eine im Inneren der Schale (11a-11b) integrierte Zentraleinheit (15); und
- eine Gruppe von Sensoren (16a-16h), die im Bereich der linken (14) und rechten (13) Armlehne angeordnet sind, wobei die Sensoren (16a-16h) über Öffnungen in der Schale (1la-1lb) mit der Zentraleinheit (15) verbunden sind;
**dadurch gekennzeichnet, dass** die Schale (1la-1lb) aus zwei lösbaren Teilen (20-21) hergestellt ist:
- einem ersten Teil (20), der die rechte Armlehne (13) bildet, den Sitz (12) und mindestens vier weitere Sensoren (16a- 16g) trägt und die Zentraleinheit (15) enthält; und
- einen zweiten Teil (21), der die linke Armlehne (14) bildet und mindestens einen Blutdrucksensor (16h) trägt, so dass der Aufbau der Vorrichtung für medizinische Fernkonsultation durch festes Verbinden der beiden Teile (20-21) der Schale (11a-11b) und durch Anschließen des mindestens einen Blutdrucksensor (16h) an die Zentraleinheit (15) durchführbar ist.

2. Vorrichtung für medizinische Fernkonsultation nach Anspruch 1, wobei die Vorrichtung ebenfalls einen Bildschirm (22a) umfasst, der bestimmt ist, gegenüber dem Sitz (12) befestigt zu sein, ohne mechanisch mit dem Sitz (12) verbunden zu sein.

3. Vorrichtung für medizinische Fernkonsultation nach Anspruch 1, wobei die Schale (1la-1lb) ebenfalls einen dritten Teil (26) aufweist, der einen Bildschirm (22b) integriert, so dass die Verbindung der drei Teile (20, 21, 26) der Schale (1la-1lb) eine Kabine mit dem gegenüber dem Sitz (12) platzierten Bildschirm bildet.

4. Vorrichtung für medizinische Fernkonsultation nach einem der Ansprüche 1 bis 3, wobei der erste Teil (20), der die rechte Armlehne (13) bildet, eine Waage (16a) und/oder einen Höhensensor (16b) trägt.

5. Vorrichtung für medizinische Fernkonsultation nach einem der Ansprüche 1 bis 4, wobei der erste Teil (20), der die rechte Armlehne (13) bildet, einen Sensor der Körperzusammensetzung (16c), ein Oximeter (16d), ein Audiometer (16e), ein Elektrokardiogramm (16f), ein Ultraschallgerät (16g), ein Otoskop, ein Stethoskop, ein Inspirationsmessgerät, ein Dermatoskop, ein Ultraschallgerät und ein Sehscanner trägt.

6. Vorrichtung für medizinische Fernkonsultation nach Anspruch 5, wobei der Sensor der Körperzusammensetzung (16c) einem Fuß-/Hand-Sensor entspricht.

7. Vorrichtung für medizinische Fernkonsultation nach Anspruch 2, wobei die Vorrichtung für Fernkonsultation (10a-10b) ebenfalls ein Zugangsterminal (27) aufweist, das bestimmt ist, am Eingang eines Raums befestigt zu sein, in dem der Bildschirm (22a-22b) und der Sitz (12) installiert sind, wobei das Zugangsterminal (27) Identifikationsmittel und einen Temperatursensor aufweist.

8. Vorrichtung für medizinische Fernkonsultation nach einem der Ansprüche 1 bis 7, wobei die Schale mechanische Unverwechselbarkeitselemente (23) umfasst, die zwischen den beiden Teilen (20-21) der Schale (1la-1lb) zusammenwirken.

9. Vorrichtung für medizinische Fernkonsultation nach einem der Ansprüche 1 bis 8, wobei die Schale (1la-1lb) eine hinter dem Sitz (12) des ersten Teils (20) der Schale (1la-11b) eingerichtete Zugangsklappe (24) aufweist.

10. Verfahren zur Installation der Vorrichtung für medizinische Fernkonsultation nach einem der Ansprüche 1 bis 9, wobei das Verfahren die folgenden Schritte aufweist:
- Bewegen des ersten Teils (20) der Schale (11a-11b) in einen Raum;
- Bewegen des zweiten Teils (21) der Schale (11a-11b) in den Raum;
- Befestigen des zweiten Teils (21) am ersten Teil (20) der Schale (11a-11b);
- Anschließen des mindestens einen Blutdrucksensors (16h) an die Zentraleinheit (15);
- Integration eines Bildschirms (22a-22b) in den Raum; und
- Anschließen des Bildschirms (22a-22b) an die Zentraleinheit (15).

## Claims

1. A device for remote medical consultation (10a-10b) having:
- a shell (11a-11b) supporting a seat (12) and forming left (14) and right armrests (13);
- a central unit (15) incorporated inside the shell (11a-11b); and
- a set of sensors (16a-16h) arranged at the left (14) and right (13) armrests, the sensors (16a-16h) being connected to the central unit (15) through openings in the shell (11a-11b);
**characterised in that** the shell (11a-11b) is produced in two removable parts (20-21):
- a first part (20) forming the right armrest (13), supporting the seat (12) and at least four other sensors (16a-16g) and incorporating the central unit (15); and
- a second part (21) forming the left armrest (14) and supporting at least one blood pressure sensor (16h) such that the installation of the remote medical consultation device can be carried out by joining together the two parts (20-21) of the shell (11a-11b) and by connecting the at least one blood pressure sensor (16h) on the central unit (15).

2. The remote medical consultation device according to claim 1, wherein the device also has a screen (22a) intended to be fixed facing the seat (12) without being mechanically connected to the seat (12).

3. The remote medical consultation device according to claim 1, wherein the shell (11a-11b) also comprises a third part (26) incorporating a screen (22b) such that the assembly of the three parts (20, 21, 26) of the shell (11a-11b) forms a cabin with the screen placed facing the seat (12).

4. The remote medical consultation device according to one of claims 1 to 3, wherein the first part (20) forming the right armrest (13) supports a weighing scale (16a) and/or a height sensor (16b).

5. The remote medical consultation device according to one of claims 1 to 4, wherein the first part (20) forming the right armrest (13) supports a body composition sensor (16c), an oximeter (16d), an audiometer (16e), an electrocardiogram (16f), an ultrasound device (16g), an otoscope, a stethoscope, a spirometer, a dermatoscope, an ultrasound device and a vision scanner.

6. The remote medical consultation device according to claim 5, wherein the body composition sensor (16c) corresponds to a hand-to-foot sensor.

7. The remote medical consultation device according to claim 2, wherein the remote consultation device (10a-10b) also comprises an access terminal (27), intended to be fixed at the entrance to the space in which the screen (22a-22b) and the seat (12) are installed, the access terminal (27) comprising identification means and a temperature sensor.

8. The remote medical consultation device according to one of claims 1 to 7, wherein the shell comprises mechanical poka-yoke devices (23) cooperating between the two parts (20-21) of the shell (11a-11b).

9. The remote medical consultation device according to one of claims 1 to 8, wherein the shell (11a-11b) comprises an access hatch (24) arranged behind the seat (12) of the first part (20) of the shell (11a-11b).

10. A method for installing the remote medical consultation device according to one of claims 1 to 9, the method comprising the following steps:
- moving the first part (20) of the shell ( 11a-11b) into a room;
- moving the second part (21) of the shell (11a-11b) into the room;
- fixing the second part (21) on the first part (20) of the shell (11a-11b);
- connecting the at least one blood pressure sensor (16h) on the central unit (15);
- incorporating a screen (22a-22b) in the room; and
- connecting the screen (22a-22b) to the central unit (15).
